# EUROPEAN PATENT SPECIFICATION

(11) **EP 2 103 349 B1**
(45) Date of publication and mention of the grant of the patent: **07.09.2016**
(21) Application number: 07850666.4
(22) Date of filing: 15.12.2007
(51) Int. Cl.: B01J 35/02, B01J 37/03, A61L 9/00, A61L 9/01, B01J 27/18, C02F 1/50, C02F 1/72, D06M 11/46, D06M 11/71

(54) **STAIN-RESISTANT MATERIAL SYNTHESIZED BY REPRECIPITATION METHOD AND HAVING WEATHER RESISTANCE, AND PROCESS FOR PRODUCTION THEREOF**
NACH DEM REPRÄZIPITATIONSVERFAHREN SYNTHETISIERTES UND WITTERUNGSBESTÄNDIGES FLECKENUNEMPFINDLICHES MATERIAL UND HERSTELLUNGSVERFAHREN DAFÜR
MATIÈRE ANTITACHE ET RÉSISTANTE AUX INTEMPÉRIES PRODUITE PAR UN PROCÉDÉ DE REPRÉCIPITATION ET PROCÉDÉ DE PRODUCTION DE CETTE MATIÈRE

(30) Priority: 15.12.2006 JP 2006339152
(43) Date of publication of application: 23.09.2009
(73) Proprietor: National Institute of Advanced Industrial Science and Technology, Tokyo 100-8921 (JP); FUJIKURA KASEI CO., LTD., Itabashi-ku Tokyo 174-0046 (JP)
(72) Inventor: OHASHI, Fumihiko, Nagoya-shi Aichi 463-8560 (JP); SHIBAHARA, Atsushi, Kitakatsushika-gun Saitama 3400203 (JP)
(74) Representative: V.O.
(86) International application number: PCT/JP2007/074174
(87) International publication number: WO 2008/072747

(56) References cited:
- JP-A- 02 188 415
- JP-A- 10 244 166
- JP-A- 11 171 515
- JP-A- 11 267 519
- JP-A- 2004 057 878
- JP-A- 2005 289 778
- US-A- 6 090 736
- US-B1- 6 180 548

## Description

### TECHNICAL FIELD

The present invention relates to a stain-resistant material (also described as an environmental purification material) comprising a calcium phosphate-related compound that has been precipitated and supported on the surface of a titanium oxide substrate by a reprecipitation method, and also relates to a process of producing this stain-resistant material. More particularly, the present invention relates to a novel stain-resistant material that, in comparison with heretofore known materials, is equipped with a higher degradation activity accomplished by a more precise control of the composition, particle size distribution, and morphology of the product. The present invention further relates to a process of producing this novel stain-resistant material.

The present invention provides a novel stain-resistant material that is synthesized by a reprecipitation method and that has a high specific surface area and exhibits an excellent water resistance, heat resistance, corrosion resistance, ion-exchange capacity, and adsorption capacity. In addition, the present invention provides a novel environmental purification material comprising a calcium phosphate-related compound uniformly coated on the surface of a substrate, wherein this novel environmental purification material can be deployed, for example, by addition to or kneading into organic fibers and plastics, and can be used in applications that utilize the high functionality of the aforementioned stain-resistant material, for example, as an adsorbent for harmful pollutants, as a deodorizer, as a catalyst support, as a humidity modulating material that autonomically controls the humidity in a living environment, e.g., within a room or vehicle, or as an environmental purification material for carrying out, e.g., odor removal, degradative removal of airborne pollutants or dirt, waste water treatment, water purification treatment, the disinfection or algicidal treatment of water, and so forth.

### BACKGROUND ART

Odor in household and industrial spaces and pollution by pollutants such as automobile exhaust gases have become severe problems in recent years. In addition, there is a widespread worsening of water pollution by, for example, industrial waste water and domestic sewage, particularly with regard to the pollution of water sources by golf course agrichemicals and the organochlorine solvents refractory to treatment by water treatment methods such as the activated sludge method in current practice. The resulting environmental pollution is a very serious social problem.

One method often used to remove odor or airborne pollutants is to absorb or adsorb the components thereof in an absorption solution, e.g., acid or alkali or with an adsorbent. However, treatment of the waste solution or spent adsorbent is a problem with this approach and creates the risk of causing secondary pollution. In another method the odor is masked using a fragrance or air freshener. However, there are drawbacks to this type of method, such as a risk that the odor of the fragrance or air freshener will transfer to food products and a risk of harm by the odor of the fragrance or air freshener itself.

The exposure of titanium oxide, which has a photocatalytic activity, to light results in the production of electrons, which exhibit a strong reducing activity, and holes, which exhibit a strong oxidizing activity, and molecular species that come into contact with the titanium oxide are then degraded by a redox activity. This activity by titanium oxide, i.e., the redox activity exhibited by a photocatalyst, can be utilized to degrade and eliminate, for example, organic solvents dissolved in water, environmental pollutants such as agrichemicals and surfactants, and airborne pollutants and odors.

The use of this photocatalytic activity has the following advantages: this method uses just titanium oxide and light; the reaction product is harmless carbon dioxide; repetitive use is a possibility; the reaction conditions, e.g., temperature, pH, gas atmosphere, toxicity, and so forth are less restricted than for microbial-based biological treatments; and compounds refractory to treatment by biological methods, such as organohalogen compounds and organophosphorus compounds, can be easily degraded and eliminated.

However, titanium oxide powder as such has mainly been used as the photocatalyst in much of the extant research and development related to means and methods of degrading and eliminating organics using titanium oxide photocatalysts. Handling and deployment were problematic as a result, for example, recovery of the photocatalyst after use was quite difficult and there was little possibility for commercialization. This resulted in attempts to use titanium oxide photocatalysts by compounding them into an easy-to-handle medium such as fiber or plastic.

However, the powerful photocatalytic activity exhibited by titanium oxide caused the degradation of not just harmful organics and environmental pollutants, but also the fiber or plastic itself, and the resulting very easy deterioration made it impossible to use titanium oxide photocatalysts through their addition to or compounding into fibers or plastics. Moreover, when a titanium oxide photocatalyst has been deployed as an antibacterial or antifungal material, it has been difficult for the bacteria or fungi to attach to the photocatalyst, for example, under running water, which has impaired the manifestation of the antibacterial or antifungal activity and has resulted in a poor efficiency.

Several ameliorative technologies have been introduced in view of the problems cited above. Thus, the following, for example, have been introduced as prior art: an environmental purification material comprising calcium phosphate coated on the surface of titanium dioxide using a pseudo-body solution (refer, for example, to Patent References 1 and 3); a coating composition containing calcium phosphate-coated titanium dioxide (refer to Patent Reference 2); and a composite material comprising calcium phosphate single crystals grown on the surface of titanium dioxide using a pseudo-body solution (refer to Patent Reference 4).

However, when evaluating these photocatalytic composite materials, one notes that precise control of the reaction conditions is required since calcium phosphate is being coated on the titanium dioxide by imitating the biologically occurring bone-forming reaction through the use of a pseudo-body solution; in practice, however, this control is difficult to achieve. In addition, a long period of time is required for crystal growth, which results in a poor production efficiency and works against mass production. Thus, the synthesis of these composite materials with a good industrial efficiency and at a good reproducibility has been quite difficult and their use has thus been strongly limited.

In addition, the pseudo-body solution used here contains a large excess of components, such as sodium chloride, that function as so-called synthesis reaction assistants, but which are not present in the composition of the synthetic composite material; this creates a requirement during production of the target composite material for large amounts of pure water for rinsing (decantation) after the composite material has been synthesized. When rinsing is inadequate, these components remain in the composite material and cause, for example, a reduction in quality. This rinsing requirement thus creates substantial problems with regard to production efficiency and quality, including the generation of large amounts of rinse waste solution.

Patent Reference 1: Japanese Patent 3,493,393
Patent Reference 2: Japanese Patent Application Laid-open No. 2000-1631
Patent Reference 3: Japanese Patent 3,275,032
Patent Reference 4: Japanese Patent Application Laid-open No. 2004-58050 JP2005-289778 discloses a similar reprecipitation process giving calcium phosphate-related compounds supported on titania.

Given such circumstances and considering the prior art described above, the present inventors carried out extensive and intensive investigations with the goal of developing a novel environmental purification material that can be conveniently produced at high efficiencies, that has a high adsorptivity and a high degradative action, and that exhibits a strong degradative performance. As a result, it was discovered that - by using a reprecipitation method that can efficiently synthesize large amounts of a porous calcium phosphate-related compound that, while lacking activity as a photocatalyst, has the ability to adsorb, for example, various microorganisms, to uniformly coat this calcium phosphate-related compound over a titanium oxide substrate - the photocatalytic functionality possessed by the titanium oxide substrate is not impaired, its durability can be raised, and use by the addition to, for example, a medium such as organic fibers and plastics, is made possible. The present invention was achieved based on these discoveries.

### DISCLOSURE OF THE INVENTION

Being developed based on a consideration of the points cited above, the present invention has as an object the introduction of a novel environmental purification material that can effectively, economically, and safely carry out environmental purification, e.g., odor removal, degradation and removal of airborne pollutants and dirt, waste water treatment, water purification treatment, antibacterial and antifungal activity, and so forth; that when deployed by the addition, for example, by compounding or kneading, to a medium such as organic fiber or plastic, does not cause deterioration of the medium; and that also exhibits an excellent behavior from the standpoint of durability. A further object of the present invention is to provide a processed article of this novel environmental purification material. Another object of the present invention is a process of producing a novel environmental purification material by precipitating and supporting a porous calcium phosphate-related compound by a reprecipitation method on the surface of a substrate having a surface comprising titanium oxide, wherein this coating of the porous calcium phosphate-related compound on the titanium oxide substrate surface can be carried out with very precise control of the composition, particle diameter distribution, morphology, and so forth.

The present invention, which achieves the objects cited above, comprises the following technical means, as defined in claims 1,3 and 5.

The present invention is described in additional detail in the following.

The process of the present invention uses a phosphate compound and a calcium compound as starting materials for coating a titanium oxide substrate. The reagent used as the phosphate source may be any reagent that ionizes in aqueous solution to give the phosphate ion, and organic and inorganic phosphates are suitable examples. Specific examples are phosphate compounds such as phosphoric acid, sodium hydrogen phosphate, potassium hydrogen phosphate, ammonium hydrogen phosphate, sodium pyrophosphate, sodium tripolyphosphate, sodium hexametaphosphate, and so forth.

One or two or more of these phosphate compounds can be used in combination. Any compound that acts to provide the calcium ion in aqueous solution may be used as the source of the calcium that bonds with the aforementioned phosphate compound, and suitable specific examples in this regard are calcium compounds such as calcium chloride, calcium nitrate, calcium sulfate, calcium carbonate, calcium hydroxide, and so forth. One or two or more of these calcium compounds may be used in combination.

Neither the phosphate source nor the calcium source is limited to the compounds provided above, and species having the same effect as the preceding can be similarly used. In addition, separate aqueous solutions containing the phosphate ion and calcium ion can be prepared and used. However, there is no limitation to the preceding, and a solution prepared by dissolving apatite and/or a crystalline or noncrystalline calcium phosphate compound in an acidic solution can also be used as the phosphate source and calcium source. In the context of the present invention, the "calcium phosphate-related compound" is defined as meaning at least one selection from calcium phosphates made from the aforementioned phosphate source and calcium source.

Viewed from the standpoint of a high photocatalyst performance, the crystal form of the titanium oxide substrate (carrier) used in the present invention is preferably anatase. Rutile, brookite, and noncrystalline species are less preferred due to their low photocatalyst activity, but these can be used as appropriate. In addition, the particle diameter of the titanium oxide particles may be of any size, but a small, submicron size is preferred when incorporation into an organic fiber or plastic is contemplated.

The stain-resistant material of the present invention is prepared substantially by dispersing titanium oxide, or a substrate having a surface coated with titanium oxide, in an acidic starting solution that contains the aforementioned phosphate ion and calcium ion and thereafter precipitating and supporting a porous calcium phosphate-related compound on the surface of the preceding by a reprecipitation method by adding an alkaline solution.

More specifically, in order to coat, for example, titanium oxide particles, a mixed solution of the calcium compound and phosphate ion having a concentration of 1 mmol/L to 1000 mmol/L is prepared by dissolving the preceding starting materials, for example, a phosphate compound and a calcium salt compound, in an acidic aqueous solution. The pH of the aqueous solution at this time is acidic and is preferably a pH at which the phosphate ion and calcium ion are dissolved, wherein a suitable pH is desirably about 1 to 4.

The calcium/phosphorus molar ratio in the solution at this point is suitably about 0.5 to 2.0. This is because by-products, e.g., calcium oxide and so forth are produced in large amounts when there is a significant departure from this molar ratio. For example, when sparingly soluble hydroxyapatite is employed as the starting material, this hydroxyapatite can be used for the starting solution for the production of the inventive stain-resistant material having weather resistance and synthesized by a reprecipitation method, because hydroxyapatite readily dissolves when the solution pH is made 3 or less using, for example, 1 to 5 N hydrochloric acid.

In those instances in which the pH of the mixed solution departs significantly from the region given above with the goal of controlling the composition, it is also effective to have calculated the acid component in the aqueous solution in order to adjust the pH and to have added the acid component to the aqueous solution. The acid component added in order to adjust the pH in such instances is suitably, for example, hydrochloric acid, nitric acid, acetic acid, formic acid, or sulfuric acid.

The concentration of the phosphate ion and calcium ion present in the acidic solution is preferably 1 to 1000 mmol/L, more preferably 1 to 600 mmol/L, and even more preferably 1 to 400 mmol/L. At less than 1 mmol/L, too little calcium phosphate-related compound precipitates and the manifestation of the effects becomes problematic.

When, on the other hand, 1000 mmol/L is exceeded, this can cause the quantity of the reagent added to provide an acidic solution, e.g., hydrochloric acid, nitric acid, sulfuric acid, and so forth to become excessive and can also cause the quantity of reagent added for neutralization, e.g., sodium hydroxide, potassium hydroxide, ammonium hydroxide (aqueous ammonia), and so forth in the neutralization step, vide infra, to become excessive. This results in the presence of large amounts of ions not required for production of the calcium phosphate-related compound, e.g., chloride ion, nitrate ion, sulfate ion, sodium ion, potassium ion, ammonium ion, and so forth, which then necessitates decantation for their removal and thus requires large amounts of pure water.

The titanium dioxide powder is weighed out in order to provide a proportion therefor of 99.9 to 50 weight% with respect to the calculated calcium phosphate-related compound precipitation from the starting solution, and is then added to the starting solution with stirring. There is no particular requirement on the stirring method, and stirring may be carried out using, for example, a magnetic stirrer or a motor-driven stirring rod. In order as may be necessary to improve the state of the dispersion, a device such as an homogenizer, bead mill, or ultrasound cleaner may be used.

A calcium phosphate-related compound is then precipitated and coated onto the titanium dioxide surface by gradually adding an alkaline solution to the acidic starting solution in which the titanium dioxide is dispersed. Precipitation of the calcium phosphate-related compound on the titanium dioxide surface is brought about by adjusting the pH into the neighborhood of 9 to 10 by the dropwise addition of the alkaline solution at a rate of 0.1 to 5 mL/minute while stirring the titanium dioxide suspension.

Sodium hydroxide, potassium hydroxide, aqueous ammonia, and so forth, are suitable examples for this alkaline solution that is added dropwise during the calcium phosphate-related compound production step. The calcium phosphate-related compound is of course also produced from a pH for the solution during the mixing phase in the pH 6.5 region around neutrality. The reaction temperature range at this time is suitably in the vicinity of room temperature, but may be in the range from 5 to 40°C. The reaction time is about 0.1 to 6 hours. Reprecipitation is favorably carried out by a method that as much as possible avoids evaporation of the water fraction in the reaction suspension; for example, a heating mantle can be used with an installed condenser.

After the completion of the reaction, the resulting product, either as such or after rinsing a plurality of times with pure water, is dried to yield a stain-resistant material that is an environmental purification material in which the surface of titanium oxide is coated with a calcium phosphate-related compound. Because the resulting product is an inorganic compound, it has a high heat resistance and can therefore be dried under relatively severe conditions, and drying is suitably performed at a temperature of 40 to 100°C under ambient pressure.

In this case, after the completion of the reaction the suspension may also be recovered as a paste using a centrifugal separator or a semipermeable membrane. The proportion of the calcium phosphate-related compound in the stain-resistant material obtained in the described manner is preferably 0.1 to 50 weight% and more preferably is 1 to 30 weight%. The appearance of the effects becomes problematic at less than 0.1 weight%. When, on the other hand, 50 weight% is exceeded, the appearance of the photocatalyst effect due to the titanium oxide in the stain-resistant material can be problematic.

The above-described method produces a stain-resistant material that is an environmental purification material in which titanium dioxide is coated with a calcium phosphate-related compound, wherein a characteristic feature of this stain-resistant material is that the average particle diameter thereof in the volumetric distribution from the light-scattering intensity distribution is within 50 nm to 1000 nm. Another feature of this stain-resistant material is that the properties of the coating film can be varied by adjusting the concentration of the applied phosphate ion solution and calcium ion solution.

The environmental purification material of the present invention exhibits an even higher rate of chemical substance oxidative degradation and also an even better antibacterial and algicidal activity when a metal, e.g., platinum, rhodium, ruthenium, palladium, silver, copper, zinc, and so forth is supported at the film surface or within the framework of the calcium phosphate-related compound film coating the titanium oxide.

The porosity, film thickness, and configuration of the calcium phosphate-related compound at the surface of the environmental purification material of the present invention can be adjusted by changing the chemical composition, temperature, and immersion time. A domain-configured calcium phosphate-related compound or a thin film of the calcium phosphate-related compound is produced at the substrate surface at a low phosphate and calcium content and a short reaction time. A thicker film of the calcium phosphate-related compound is obtained by raising the phosphate and calcium content and lengthening the reaction time.

The surface of the inventive environmental purification material obtained in the described manner is coated by a film of a calcium phosphate-related compound that is not active as a photocatalyst. In addition, because this calcium phosphate-related compound film can adsorb, for example, proteins, amino acids, bacteria, viruses, and so forth, the calcium phosphate-related compound surface film can adsorb, for example, waterborne and airborne bacteria. In addition, this calcium phosphate-related compound film has pores at the surface and thus provides a configuration in which the photocatalytically active titanium oxide is exposed at the bottom of these pores.

As a consequence, artificial light, e.g., from a fluorescent lamp, incandescent lamp, blacklight, UV lamp, mercury lamp, xenon lamp, halogen lamp, metal halide lamp, and so forth and sunlight can reach this exposed region. In addition, the protein, amino acids, bacteria, viruses, and so forth adsorbed by the calcium phosphate-related compound film can be rapidly and continuously degraded and removed by the redox activity of the electrons and holes produced by the titanium oxide upon exposure to light.

In addition, when this environmental purification material is deployed by kneading into a medium such as a fiber or resin, the region in contact with such an organic compound material is a ceramic that is inactive as a photocatalyst. As a result, airborne pollutants, e.g., odors, nitrogen oxides, sulfur oxides, and so forth and organic compounds that pollute the environment, e.g., water-dissolved organic solvents and agrichemicals can be adsorbed and can be rapidly and continuously degraded and removed by the redox activity of the electrons and holes produced by the titanium oxide upon exposure to sunlight or artificial light, e.g., from a fluorescent lamp, incandescent lamp, blacklight, UV lamp, mercury lamp, xenon lamp, halogen lamp, metal halide lamp, and so forth without degradation occurring to the aforementioned organic compounds.

In addition, the environmental purification material of the present invention can be used just by exposure to light in a low cost, energy-conserving, and maintenance-free manner. Moreover, the environmental purification effects, e.g., degradative removal of organic compounds, antibacterial/antifungal activity, and so forth, can be further strengthened by the catalytic activity provided by using environmental purification material according to the present invention in which a metal, e.g., platinum, rhodium, ruthenium, palladium, silver, copper, iron, zinc is supported in the interior framework of the calcium phosphate-related compound coated on the titanium oxide particles.

A wide variety of organic fibers and plastics can be used as the fiber or resin medium with the environmental purification material according to the present invention, for example, of acrylic resin, polycarbonate, saturated polyester, thermoplastic elastomer, urea resin, melamine resin, unsaturated polyester resin, silicone resin, polyimide, polyethylene, nylon, polyvinyl chloride, polyvinylidene chloride, polyester, polypropylene, polyethylene oxide, polyethylene glycol, polyethylene terephthalate, silicon resin, polyvinyl alcohol, vinyl acetal resin, polyacetate, ABS resin, epoxy resin, vinyl acetate resin, cellulose, cellulose derivatives, polyamide, polyurethane, polystyrene, urea resin, fluororesin, polyvinylidene fluoride, phenolic resin, celluloid, chitin, starch sheet, and so forth, as well as copolymers of the preceding.

The novel environmental purification material according to the present invention, which is produced by a reprecipitation method, exhibits an excellent corrosion resistance and the excellent water resistance and heat resistance inherent to inorganic compounds. As a result, this novel purification material can be used in a broad range of industrial fields, e.g., for the degradative removal of airborne pollutants, e.g., odors, smoke such as tobacco smoke, NOx, SOx, for the degradative removal of organic compounds such as organic solvents and agrichemicals dissolved in water, for waste water treatment and water purification treatment, to prevent staining, as an antibacterial and antifungal, to prevent nosocomial inflections by, e.g., MRSA, as a catalyst carrier, as a humidity modulating material that autonomically controls the humidity in living environments, e.g., in rooms and vehicles, as a water-purification filter and as a microcapsule for chemicals that utilizes its unique shape, and is also very effective for purification of the environment.

In addition, the aforementioned titanium oxide is also used in, for example, coatings, cosmetics, and tooth powders, and is also approved as a food additive. It is nontoxic, safe, and inexpensive and also has an excellent weather resistance and durability. In addition, because the aforementioned calcium phosphate-related compound film applied by a reprecipitation method is inactive as a photocatalyst, even when the purification material is deployed by the addition by kneading into a medium such as an organic fiber or plastic, the medium is not subjected to deterioration and the purification activity of the purification material can be maintained on a long-term basis.

As a result, through its addition to a coating or a medium such as an organic fiber or plastic, the environmental purification material according to the present invention can be deployed in an extremely broad range of applications, i.e., not just for the deodorization of automotive interiors, living rooms, kitchens, and bathrooms, for waste water treatment, or for cleaning pools or impounded water, but also to prevent the growth of bacteria and fungi and prevent the spoilage of food products. Moreover, the environmental purification material according to the present invention does not use a harmful material such as a chemical or ozone and simply by exposure to light, e.g., natural light or light from an electric lamp can be safely used on a long-term basis in a low cost, energy-conserving, and maintenance-free manner.

In addition, the environmental purification material according to the present invention can be produced by a reprecipitation method after the titanium dioxide substrate has been introduced into an acidic solution that contains both the phosphate ion and the calcium ion. At this time, for example, the size of the pore diameters in the surface and the density of the pore distribution can be precisely adjusted by changing the reaction temperature and time and the composition of the precursor acidic solution for the calcium phosphate-related compound.

For example, an apatite photocatalyst obtained by supporting apatite on a titanium oxide surface using a pseudo-body solution is known as prior art for the present invention. However, when control of the particle diameter distribution and morphology of the material is considered, it has been difficult with this prior material to control the particle diameter distribution and the morphology and there has been scatter or dispersion in the particle diameter distribution and morphology and there have also been limits on the ability to raise the degradation activity. In addition, when the production method and production efficiency are considered, crystal growth is slow with the prior material and this has placed limits on production at the industrial product level.

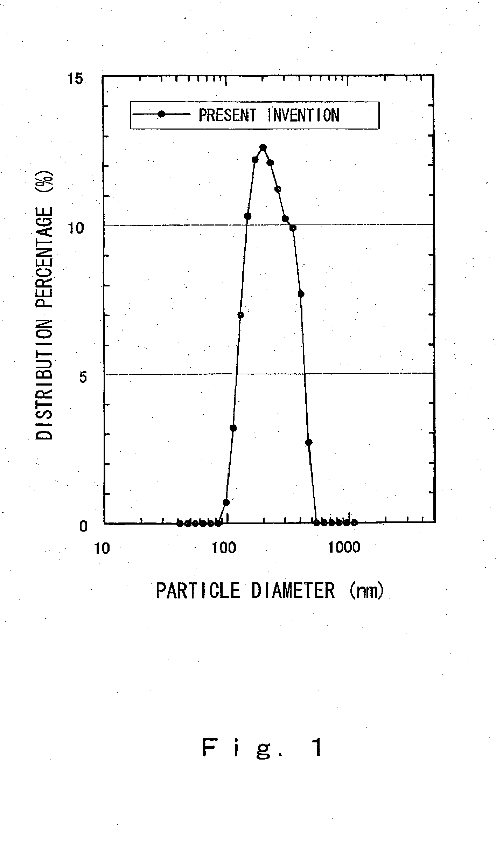

In contrast, the material of the present invention, in which a calcium phosphate-related compound has been reprecipitated and supported using a reprecipitation method, is very good with regard to productivity and quality and is capable of convenient and rapid mass production. With reference to the resulting product, a highly precise control of the composition, particle diameter distribution, and morphology of the product is achieved that has not been possible with heretofore known materials. In particular, the particle distribution is clustered or converged (refer to Figure 1), as a result of which the degradation activity is also controlled at the same time and the activity is boosted.

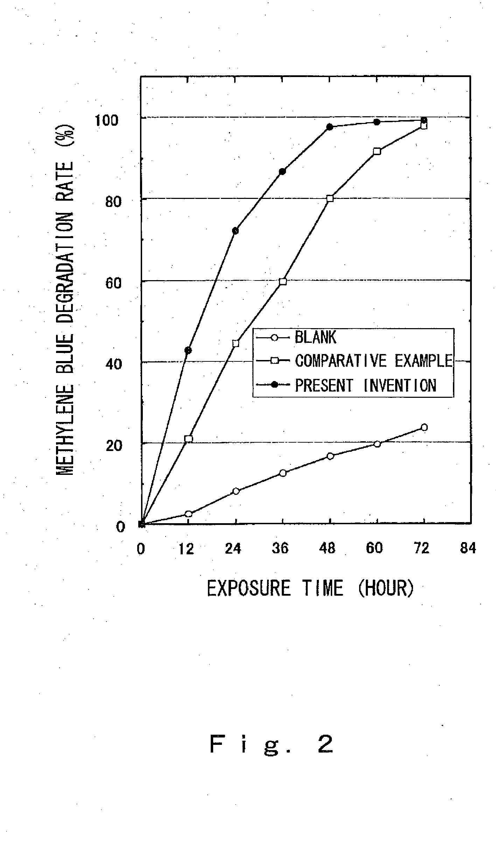

The inventive stain-resistant material having weather resistance and synthesized by a reprecipitation method, has been shown to be much better in terms of performance and functionality than the heretofore known apatite photocatalyst (refer to Examples 1 and 2). In addition, coating films obtained by forming a composite between a resin and a powder of the stain-resistant material of the present invention have been shown to have a higher performance than coating films obtained by forming a composite between a resin and the heretofore known apatite photocatalyst (Refer to Figures 2, 3, and 4).

The following effects are accrued by the present invention.
(1) The present invention can provide a stain-resistant material that has a particle structure comprising a porous calcium phosphate-related compound uniformly supported on the surface of a titanium oxide substrate by reprecipitation on the surface of the titanium oxide substrate by a reprecipitation method; that has a particle diameter controlled into a prescribed nanosize range; and that has a high degradation activity.
(2) With the environmental purification material obtained in accordance with the present invention, the calcium phosphate-related compound coating the surface is porous, and as a result the titanium oxide at the bottom of the pores is exposed and light reaches the titanium oxide substrate in this region. In addition, odors, airborne pollutants, and organic compounds that pollute the environment, e.g., organic solvents and agrichemicals dissolved in water can be easily adsorbed and degraded and eliminated due to the adsorption activity of the calcium phosphate-related compound and the redox activity of the electrons and holes produced by exposure to light.
(3) In addition, since the calcium phosphate-related compound portion of the aforementioned material is inactive as a photocatalyst, in those instances in which the environmental purification material is deployed by addition by, for example, incorporation into a medium such as organic fiber or plastic, the calcium phosphate-related compound has a protective effect and degradation of the fiber or plastic itself is inhibited. This enables continuous use with long-term retention of the effects.
(4) The calcium phosphate-related compound described in the preceding has the ability to adsorb, for example, microorganisms and so forth, and therefore adsorbs, for example, pollutants and so forth. The titanium oxide substrate can carry out a reliable and efficient destruction and degradation due to the strong oxidizing power produced by the titanium oxide when it is exposed to light.

### BRIEF DESCRIPTION OF THE DRAWINGS

Figure 1 is the particle size distribution curve (volumetric basis from the light scattering intensity distribution) for an environmental purification material as an example of the present invention in which coating by a calcium phosphate-related compound was performed by a reprecipitation method according to the present invention;
Figure 2 shows the results of a methylene blue decolorization test on a calcium phosphate-related compound-coated environmental purification material coating film according to an example of the present invention;
Figure 3 shows the results of a weathering resistance test based on color difference for a calcium phosphate-related compound-coated environmental purification material coating film according to an example of the present invention;
Figure 4 shows the results of a weathering resistance test based on gloss retention rate for a calcium phosphate-related compound-coated environmental purification material coating film according to an example of the present invention; and
Figure 5 is the particle size distribution curve (volumetric basis from the light scattering intensity distribution) for an environmental purification material as a comparative example for the present invention in which coating by a calcium phosphate-related compound was performed by a conventional method.

### BEST MODE FOR CARRYING OUT THE INVENTION

The present invention is specifically described below based on examples, but the present invention is in no way limited by the examples that follow.

### Example 1

1.8 g hydroxyapatite powder was added to and dissolved in an acidic solution of 13 mL 5 N hydrochloric acid mixed with 970 mL deionized water. The pH at this time was 1.5. 10.0 g titanium dioxide powder was added to this acidic solution and thorough dispersion was carried out by treating with ultrasound. Then, while this suspension was being stirred, 14 mL 25 weight% aqueous ammonia solution was added dropwise at a rate of 2 mL/minute and a calcium phosphate-related compound was reprecipitated. The pH of the final reaction suspension was 9.7 and the temperature was 24°C.

After the completion of the reaction, a thorough washing was carried out using a centrifugal separator. This was done in order to remove the calcium phosphate-related compound that was not bonded to the surface of the substrate. The difference between the specific gravity of the titanium dioxide substrate and the produced calcium phosphate-related compound makes possible their separation and purification by centrifugal separation. This was followed by drying at ambient pressure and 40°C in an electric drier to obtain a calcium phosphate-related compound-coated environmental purification material (stain-resistant material).

Chemical composition analysis by fluorescent x-ray analysis (Rayny EDX-700HS from Shimadzu Corporation) was carried out in order to examine the chemical composition of the calcium phosphate-related compound-coated environmental purification material obtained as described above. The results were 85.53 weight% titanium, 9.64 weight% calcium, and 3.81 weight% phosphorus as the elemental ratio, which suggested that about 15 weight% calcium phosphate-related compound was coated on the titanium dioxide substrate.

The calcium/phosphorus ratio at this time was shown to be about 1.95. In addition, the residual percentages for the phosphorus and calcium present in the supernatant after the completion of the reaction were measured by ICP analysis (Ciros 120 EOP from Spectro Analytical Instruments Incorporated). These residual percentages were shown as a result to be 0.72% and 2.76%, respectively, which demonstrated that almost all of the chemical species present in the starting solution participated in the reaction.

### Example 2

A decolorization test was run on a dyed waste solution using the stain-resistant material powder described above. For the comparative example, a material in which apatite had been coated according to a conventional method on the surface of titanium dioxide using a pseudo-body solution was employed for the reference specimen. An aqueous methylene blue solution was used as the model waste solution. Each specimen was prepared by blending the sample at 0.5 weight% into a 20 ppm aqueous methylene blue solution.

While the specimen was being stirred with a magnetic stirrer, the specimen was exposed to a blacklight BLB to provide 1 mW/cm², and after two hours the degree of decolorization of the aqueous methylene blue solution was measured using the absorbance. For the measurement, the sample, which was in a suspended state, was separated by centrifugal separation and the resulting supernatant was submitted to measurement. A digital colorimeter (miniphoto 10, Sanshin Industrial Co., Ltd., filter: 660 nm) was used for measurement of the absorbance. An evaluation as an environmental material was performed based on measurement of the percentage dye degradation.

According to the results, the percentage degradation for the blank of the only methylene blue was 0.8%, which indicated almost no degradation. The degradation percentage for the material prepared by the conventional method was 87.8%, while the environmental purification material of the present invention gave a high numerical value for the dye degradation percentage of 91.25%. Measurement of the particle size distribution was performed by a laser scattering procedure (Zetasizer 3000HSA, Malvern Instruments Ltd.). The particle size distribution curve (volumetric basis from the light scattering intensity distribution) of the inventive material obtained by a reprecipitation method is given in Figure 1. According to these results, it was shown that the particle diameter of the inventive material synthesized by a reprecipitation method was distributed in the range of 80 to 600 nm.

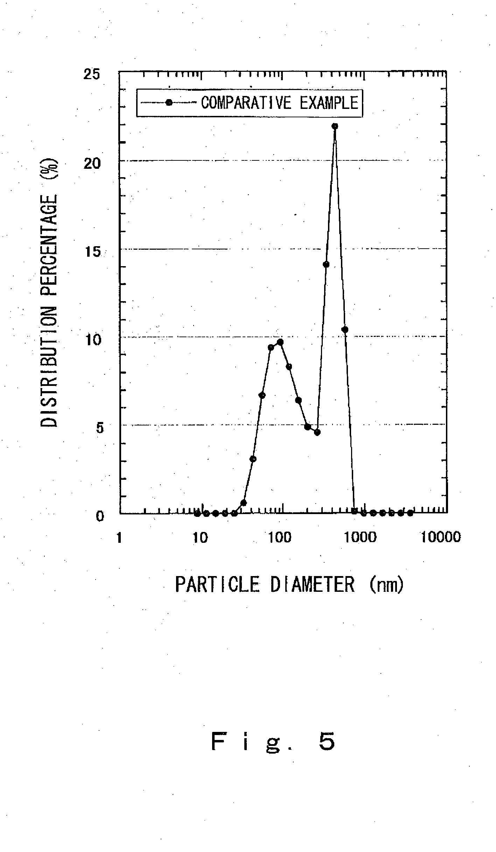

Next, the particle size distribution curve (volumetric basis from the light scattering intensity distribution) of the material obtained by the conventional method is given in Figure 5. The particle size range was shown to be about 30 to 900 nm and a doublet peak at around 90 nm and around 450 nm was observed for the particle diameter. Scatter in the particle size distribution was not seen for the environmental purification material of the present invention as compared to the material synthesized by the conventional method, and the appearance of only a singlet peak anticipates a high dispersibility when incorporated into a resin carrier as well as a high powder particle regularity of arrangement within the resin carrier and, accompanying this, the manifestation of a uniform catalytic effect and a high retention of this effect.

### Example 3

A methylene blue decolorization test was performed on a coating film obtained by forming a composite between the aforementioned environmental purification material powder and a resin. For the comparative example, a material in which apatite had been coated according to a conventional method on the surface of titanium dioxide using a pseudo-body solution was employed for the reference specimen. Polydurex G-659 (product name, solids fraction = 42%, from Asahi Kasei Chemicals Corporation) was mixed as the resin component at a resin : suspension proportion of 33 : 66 (weight ratio) with a suspension containing 1 weight% of the environmental purification material powder or 1 weight% reference specimen powder, to synthesize a suspension in which the pH had been adjusted to around pH 9 using an alkaline solution.

"Water-based Ceramitone (B15-50B(5Y5R/1) Approximate Color)" (product name, from Fujikura Kasei Co., Ltd.) was coated as a water-based primer on a 5 cm x 5 cm aluminum plate. After drying, the particular suspension of the present invention or comparative example was applied by spraying to fabricate a coating film specimen. As a pretreatment, the specimen was exposed for three hours or more using a blacklight BLB so that the coated surface received an exposure intensity of 1 mW/cm².

An acrylic resin ring (outer diameter: 45 mm, inner diameter: 40 mm, height: 30 mm) was then fixed on the coated surface using a non-water-soluble adhesive. The interior of this ring was filled with 30 mL adsorption solution (8 ppm aqueous methylene blue solution). This was followed by sealing with a glass lid (50 mm x 50 mm x 0.5 mm) and holding for twelve hours or more in the dark in order to bring about a thorough adsorption of the aqueous methylene blue solution by the coating film. After this, the adsorption solution was removed and the interior of the ring was lightly washed with distilled water; 30 mL of a 4 ppm aqueous methylene blue solution was poured in; and resealing was done with the aforementioned glass lid.

Exposure with a blacklight BLB was carried out so that the coated surface received an exposure intensity of 1 mW/cm² and the timewise change in the decolorization of the aqueous solution due to methylene blue degradation was measured using the absorbance. A digital colorimeter (miniphoto 10, Sanshin Industrial Co., Ltd., filter: 660 nm) was used for measurement of the absorbance. The results of this decolorization test are shown by the graph in Figure 2. Here, a higher percentage dye degradation is indicative of a higher photocatalytic activity.

According to the results, in the case of the coating film that contained the environmental purification material of the present invention, the percentage dye degradation after 48 hours of blacklight BLB exposure was a high percentage degradation value reaching to about 100%. In contrast to this, it was shown that this value for the blank of the only the resin component of the coating film, measured as a reference was 20% and was about 80% for the coating film that contained the material synthesized by the conventional method. The coating film of the present invention was also shown to exhibit a more rapid methylene blue degradation rate than the coating film that contained material synthesized by the conventional method.

### Example 4

Weathering resistance testing was carried out on a coating film obtained by compounding the aforementioned environmental purification material powder with a resin. For the comparative example, a material in which apatite had been coated according to a conventional method on the surface of titanium dioxide using a pseudo-body solution was employed for the reference specimen. Using an ultraviolet irradiator (EYE Super UV Tester W-151 from Iwasaki Electric Co., Ltd.), a coating film specimen fabricated by the same method as previously described was irradiated for four hours at a black panel temperature of 63°C and a humidity of 50% RH so that the coated surface received an exposure intensity of 1000 mW/cm².

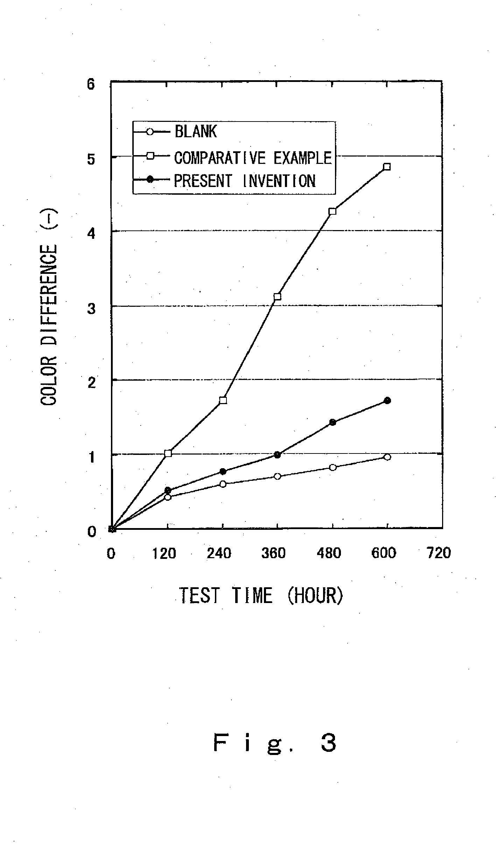

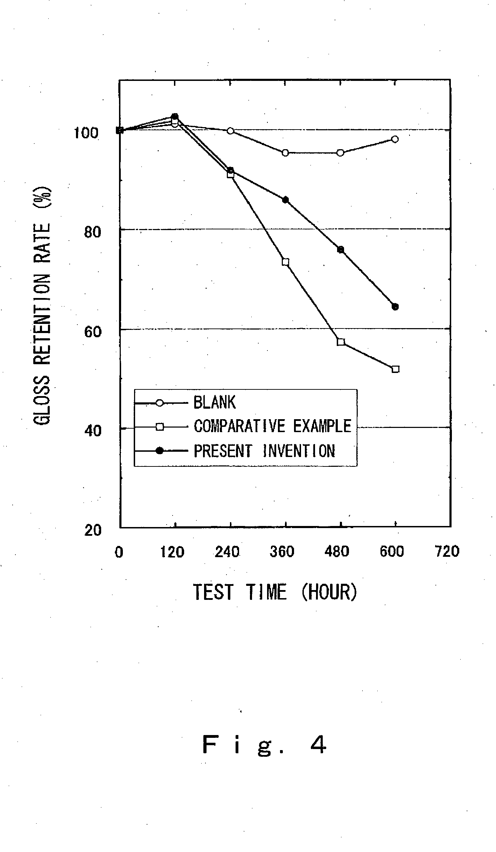

After this, the temperature in the tank was set at approximately 30°C and the humidity was set at 98% RH or more and condensation within the tank was brought about by holding for four hours. This irradiation step and condensation step were designated as 1 cycle, and the gloss retention rate and color difference were checked every 120 hours. The gloss and color difference were measured using a glossmeter and a color difference meter (SM Color Computer, Model SM-T, from Suga Test Instruments Co., Ltd.). The results are shown in Figures 3 and 4.

With regard to the color difference weathering resistance test shown in Figure 3, a procedure was used in which the color change was measured as a function of the test time using the color difference meter. Larger numerical changes in the color difference are indicative of greater color fading. For the blank of the only the resin component of the coating film, a low color fading was demonstrated after 600 hours with a color difference no greater than 1. The same trend was also seen for the coating film that contained the environmental purification material of the present invention, where the color fading was about 1.7, thus indicating an excellent color preservation. However, for the coating film in the comparative example, which contained the material synthesized by the conventional method, the numerical value for the color difference increased sharply with elapsed time and a severe deterioration in the color was seen after 600 hours.

The change in the gloss retention rate for each specimen is shown in Figure 4. The blank maintained a gloss of at least 90% even after a test time of 600 hours. With regard to the gloss retention rate for the present invention and comparative example, a trend of decline with elapsed time was seen for both specimens, but the numerical value for the gloss retention rate for the present invention was 65% after 600 hours, which was better than for the comparative example. The coating film that contained the environmental purification material of the present invention was thus shown to have a higher photoactive catalytic function and a higher weathering resistance than the coating film that contained the material synthesized by the conventional method.

### Example 5

Six solutions (lots) were prepared by adding and dissolving 0.4, 0.8, 1.2, 1.6, 2.0, or 3.0 g hydroxyapatite powder to separate acidic solutions of 970 mL deionized water mixed with from 10 to 15 mL 5 N hydrochloric acid. The pH at this point was from 1.4 to 1.6. 10.0 g titanium dioxide powder was added to each acidic solution and thorough dispersion was carried out by treating with ultrasound. Then, while the resulting suspension was being stirred, from 10 to 15 mL 25 weight% aqueous ammonia solution was added dropwise at a rate of 2 mL/minute and a calcium phosphate-related compound was reprecipitated. The pH of the final reaction suspension was from 9.4 to 9.7 and the precipitation temperature was 24°C. After the completion of the reaction, a thorough washing was carried out using a centrifugal separator. This was followed by drying at ambient pressure and 40°C in an electric drier to obtain five types of calcium phosphate-related compound-coated environmental purification materials.

Chemical composition analysis by fluorescent x-ray analysis was carried out in order to examine the chemical composition of the calcium phosphate-related compound-coated environmental purification materials obtained as described above. The results, as the elemental ratio, were as follows: 95.8, 93.1, 89.6, 86.8, 84.6, and 82.3 weight%, respectively, for titanium dioxide, and 3.33, 6.2, 9.48, 12.30, 14.48, and 16.78 weight%, respectively, for the calcium phosphate-related compound.

This suggested that the reprecipitated calcium phosphate-related compound was coated on the titanium dioxide substrate. The calcium/phosphorus ratio at this time was approximately from 1.7 to 2.0. In addition, the residual percentages for the phosphorus and calcium present in the supernatant after the completion of the reaction were measured by ICP analysis. According to the results, the residual percentages were less than or equal to the detection limits for all lots, which demonstrated that all of the chemical species present in the starting solution participated in the reaction.

### INDUSTRIAL APPLICABILITY

As has been described in the preceding, the present invention relates to a stain-resistant material having weather resistance and synthesized by a reprecipitation method, to a process of producing this stain-resistant material, and to processed articles thereof. The present invention provides a novel environmental purification material that is produced by a reprecipitation method. In this environmental purification material, the calcium phosphate-related compound film that has been precipitated by a recrystallization method and that coats the substrate surface is porous, and as a result light reaches the titanium oxide at the substrate surface by passing through the pores or through the gaps between calcium phosphate-related compound domains. As a consequence, a photocatalytic activity can be obtained that is no more than slightly inferior to that obtained in the absence of coating by the calcium phosphate-related compound film. The present invention is useful for providing a novel environmental purification material that has the ability to adsorb pollutants, e.g., microorganisms, due to the activity of the aforementioned calcium phosphate-related compound film region, and that has the ability to reliably and effectively degrade and remove the adsorbed pollutants due to the photocatalytic activity exhibited by the aforementioned titanium oxide substrate region.

## Claims

1. A process of producing a stain-resistant material having weather resistance synthesized by a reprecipitation method, wherein a calcium phosphate-related compound is supported on a surface of titanium oxide,
the process comprising:
adding and dispersing titanium oxide directly in an acidic solution that contains 1 to 1000 mmol/L phosphate ion and calcium ion provided by the dissolution of one or more source(s) of phosphate and calcium,
thereafter adding an alkaline solution to the dispersed titanium oxide solution to cause precipitation of the calcium phosphate-related compound onto the surface of the titanium oxide to thereby synthesize a stain-resistant material having a particle structure in which a porous calcium phosphate-related compound is uniformly supported on the surface of the titanium oxide, and
wherein the particle diameter distribution of the collected particles of the stain-resistant material, as measured by a laser scattering method, is in the range of 80 to 600 nm, and wherein the average particle diameter of the stain-resistant material is within 50 nm to 1000 nm in a volumetric distribution from a light-scattering intensity distribution.

2. The process according to claim 1, wherein the calcium phosphate-related compound is precipitated by adding an alkaline solution to the dispersed titanium oxide solution to adjust pH of the dispersed titanium oxide solution to be higher than pH of an acidic solution.

3. A stain-resistant material having weather resistance, the material comprising a calcium phosphate-related compound precipitated on the surface of titanium oxide by a reprecipitation method, which material has a particle structure in which a porous calcium phosphate-related compound is uniformly supported on the surface of the titanium oxide, and the particle diameter distribution of the particles, as measured by a laser scattering method, is in the range of 80 to 600 nm, wherein the average particle diameter of the stain-resistant material is within 50 nm to 1000 nm in a volumetric distribution from a light-scattering intensity distribution.

4. The stain-resistant material having weather resistance according to claim 3, wherein the proportion of the calcium phosphate-related compound in the stain-resistant material is 0.1 to 50 weight%.

5. A processed article which contains the stain-resistant material having weather resistance as defined in any one of claims 3 to 4 in an organic or inorganic emulsion, at the surface or in the interior of a resin, at the surface or in the interior of an organic fiber, or at the surface or in the interior of an inorganic carrier or support.

## Patentansprüche

1. Verfahren zur Herstellung eines schmutzabweisenden Materials mit Witterungsbeständigkeit synthetisiert durch ein Umfällungsverfahren, wobei eine Calciumphosphat-verwandte Verbindung auf eine Oberfläche von Titanoxid aufgetragen wird,
das Verfahren umfassend:
Zugeben und Dispergieren von Titanoxid direkt in eine saure Lösung, die 1 bis 1000 mmol/L Phosphation und Calciumion enthält, bereitgestellt durch das Auflösen von einer oder mehrerer Quelle(n) von Phosphat und Calcium,
danach Zugeben einer alkalischen Lösung zu der dispergierten Titanoxidlösung, um Fällen der Calciumphosphat-verwandten Verbindung auf der Oberfläche des Titanoxids zu bewirken, um auf diese Weise ein schmutzabweisendes Material zu synthetisieren mit einer Teilchenstruktur, in welcher eine poröse Calciumphosphat-verwandte Verbindung gleichmäßig auf der Oberfläche des Titanoxids aufgetragen ist, und
wobei die Teilchendurchmesserverteilung der gesammelten Teilchen auf dem schmutzabweisenden Material, wie mit einem Laserstreuverfahren gemessen, im Bereich von 80 bis 600 nm liegt, und wobei der durchschnittliche Teilchendurchmesser des schmutzabweisenden Materials innerhalb von 50 nm bis 1000 nm in einer volumetrischen Verteilung von einer Lichtstreuintensitätsverteilung liegt.

2. Verfahren nach Anspruch 1, wobei die Calciumphosphat-verwandte Verbindung durch Zugeben einer alkalischen Lösung zu der dispergierten Titanoxidlösung ausgefällt wird, um den pH-Wert der dispergierten Titanoxidlösung einzustellen, dass er höher als der pH-Wert einer sauren Lösung ist.

3. Schmutzabweisendes Material mit Witterungsbeständigkeit, das Material umfassend eine Calciumphosphat-verwandte Verbindung ausgefällt auf der Oberfläche des Titanoxids durch ein Umfällungsverfahren, wobei das Material eine Teilchenstruktur hat, in welcher eine poröse Calciumphosphatverwandte Verbindung gleichmäßig auf der Oberfläche des Titanoxids aufgetragen ist, und die Teilchendurchmesserverteilung der Teilchen, wie mit einem Laserstreuverfahren gemessen, im Bereich von 80 bis 600 nm liegt, wobei der durchschnittliche Teilchendurchmesser des schmutzabweisenden Materials innerhalb von 50 nm bis 1000 nm in einer volumetrischen Verteilung von einer Lichtstreuintensitätsverteilung liegt.

4. Schmutzabweisendes Material mit Witterungsbeständigkeit nach Anspruch 3, wobei der Anteil der Calciumphosphat-verwandten Verbindung in dem schmutzabweisenden Material zwischen 0,1 bis 50 Gew.% beträgt.

5. Verarbeiteter Gegenstand, der das schmutzabweisende Material mit Witterungsbeständigkeit wie in einem der Ansprüche 3 bis 4 definiert, in einer organischen oder anorganischen Emulsion, auf der Oberfläche oder im Inneren eines Harzes, auf der Oberfläche oder im Inneren einer organischen Faser, oder auf der Oberfläche oder im Inneren eines anorganischen Trägers oder Unterlage enthält.

## Revendications

1. Procédé de production d'une matière antitache ayant une résistance aux agents atmosphériques, synthétisée par un procédé de reprécipitation, dans lequel un composé apparenté au phosphate de calcium repose sur une surface d'oxyde de titane,
le procédé comprenant :
l'addition et la dispersion d'oxyde de titane directement dans une solution acide qui contient de 1 à 1 000 mmoles/l d'ions phosphate et d'ions calcium fournis par la dissolution d'une ou de plusieurs source(s) de phosphate et de calcium,
ensuite l'addition d'une solution alcaline à la solution d'oxyde de titane dispersé, pour provoquer la précipitation du composé apparenté au phosphate de calcium sur la surface de l'oxyde de titane, afin de synthétiser une matière antitache ayant une structure particulaire dans laquelle un composé apparenté au phosphate de calcium poreux repose uniformément sur la surface de l'oxyde de titane, et
dans lequel la distribution de diamètres des particules recueillies de la matière antitache, mesurée par une méthode de diffusion laser, se situe dans la plage de 80 à 600 nm, et dans lequel le diamètre moyen de particule de la matière antitache est dans la plage de 50 nm à 1 000 nm en une distribution volumétrique à partir d'une distribution d'intensités de diffusion laser.

2. Procédé selon la revendication 1, dans lequel le composé apparenté au phosphate de calcium est précipité par addition d'une solution alcaline à la solution d'oxyde de titane dispersé, pour ajuster le pH de la solution d'oxyde de titane dispersé afin que son pH soit plus élevé que le pH d'une solution acide.

3. Matière antitache ayant une résistance aux agents atmosphériques,
la matière comprenant un composé apparenté au phosphate de calcium précipité sur la surface d'oxyde de titane par un procédé de reprécipitation, laquelle matière a une structure particulaire dans laquelle un composé apparenté au phosphate de calcium poreux repose uniformément sur la surface de l'oxyde de titane, et la distribution de diamètres des particules, mesurée par une méthode de diffusion laser, se situe dans la plage de 80 à 600 nm, le diamètre moyen de particule de la matière antitache étant dans la plage de 50 nm à 1 000 nm en une distribution volumétrique à partir d'une distribution d'intensités de diffusion laser.

4. Matière antitache ayant une résistance aux agents atmosphériques selon la revendication 3, dans laquelle la proportion du composé apparenté au phosphate de calcium dans la matière antitache vaut de 0,1 à 50 % en poids.

5. Article traité qui contient la matière antitache ayant une résistance aux agents atmosphériques, telle que définie dans l'une quelconque des revendications 3 et 4, en une émulsion organique ou inorganique, à la surface ou à l'intérieur d'une résine, à la surface ou à l'intérieur d'une fibre organique, ou à la surface ou à l'intérieur d'un support ou d'une matière de support inorganique.
